# EUROPÄISCHE PATENTSCHRIFT

(11) **EP 0 590 259 B1**
(45) Veröffentlichungstag und Bekanntmachung des Hinweises auf die Patenterteilung: **29.10.1997**
(21) Anmeldenummer: 93111771.7
(22) Anmeldetag: 22.07.1993
(51) Int. Cl.: C07C 17/361, C07C 25/02, C07C 25/13, C07C 25/125, C09B 39/00

(54) **Verfahren zur Herstellung von Halogenaromaten**
Process for the preparation of halogenated aromatic compounds
Procédé pour la préparation de composés aromatiques halogénés

(30) Priorität: 04.08.1992 DE 4225763
(43) Veröffentlichungstag der Anmeldung: 06.04.1994
(73) Patentinhaber: BAYER AG, 51368 Leverkusen (DE)
(72) Erfinder: Lui, Norbert, Dr., D-51061 Köln (DE); Bielefeldt, Dietmar, Dr., D-40883 Ratingen (DE); Marhold, Albrecht, Dr., D-51373 Leverkusen (DE)

(56) Entgegenhaltungen:
- EP-A- 0 213 047
- EP-A- 0 427 603
- EP-A- 0 579 021
- FR-A- 2 647 106
- FR-A- 2 647 107
- US-A- 3 283 018

## Beschreibung

Die vorliegende Erfindung betrifft ein Flüssigphase-Verfahren zur Herstellung von Fluor- und/oder Chloraromaten aus aromatischen Fluor- oder Chlorameisensäureestern.

Es ist bekannt, daß sich aliphatische Fluor- und Chlorameisensäureester in flüssiger Phase in Gegenwart von Lewis-Säuren thermisch zu den entsprechenden Fluor- und Chloralkanen umsetzen lassen (siehe DE-A 2 931 777, US-A 4 814 524 und DE-C 857 350).

Versuche, diese Reaktion auf aromatische Halogenameisensäureester zu übertragen, zeigten, daß aromatische Halogenameisensäureester in anderer Weise reagieren als aliphatische Halogenameisensäureester. Aromatische Chlorameisensäureester reagieren in Gegenwart von Aromaten und Lewis-Säuren beim Erhitzen nicht unter Decarboxylierung zu Chloraromaten, sondern in einer Friedel-Crafts-Reaktion zu Phenylbenzoaten (J. Org. Chem. 22, 325 (1957)). Selbst wenn man in Abwesenheit aromatischer Lösungsmittel arbeitet, war deshalb damit zu rechnen, daß gegebenenfalls durch Decarboxylierung entstehendes Chlorbenzol sich sofort mit noch vorhandenem aromatischem Chlorameisensäureester zu Phenylbenzoaten umsetzt und Chlorbenzol nicht isoliert werden kann.

Zur Herstellung von Fluoraromaten ist vorgeschlagen worden, 1 Mol aromatischen Halogenameisensäureester in Gegenwart von 50 Mol Fluorwasserstoff und 3 Mol Trifluormethansulfonsäure in flüssiger Phase umzusetzen. Neben dem Einsatz von zwei verschiedenen Hilfsmitteln ist bei diesem Verfahren nachteilig, daß Fluoraromaten nur in Ausbeuten von 1 bis 14 % erhalten werden.

Andere Verfahren zur Herstellung von substituierten Halogenaromaten sind ebenfalls nachteilig. So liefert die direkte Halogenierung von Alkylaromaten nur schwierig auftrennbare Isomerengemische, welche das gewünschte Isomere häufig nur zu einem geringen Anteil enthalten (J. Org. Chem. 55, 5260 bis 5269 (1990)).

Die Blockierung unerwünschter Substitutionsstellen durch tertiäre Butylgruppen erlaubt die selektive Einführung von Fluor an aromatische Kerne durch Nitrieren, Reduzieren, Diazotieren, Verkochen in Gegenwart von Fluoridionen und Abspaltung der tertiären Butylgruppen (J. Chem. Soc. Perkin Trans. I 1987, 1). Nachteilig ist hier der vielstufige Verfahrensweg.

Es gibt auch eine vielstufige Synthese zur Herstellung von 2,6-Dialkylhalogenbenzolen aus 2,3-Dimethylbutadien, bei der Umsetzungen mit Dichlorcarben und Triphenylzinnwasserstoff durchzuführen sind (Synthesis, Band 6-7, S. 647 bis 649). Das benötigte 2,3-Dimethylbutadien ist schwierig zugänglich und damit teuer. Der Verfahrensweg ist auch hier umständlich und für den technischen Maßstab uninteressant.

Schließlich lassen sich aromatische Halogenameisensäureester in Gegenwart von Aluminiumoxid, das gegebenenfalls mit Edelmetallen belegt ist, in der Gasphase in Halogenaromaten umwandeln (EP-A 118 241 und 427 603).

Aus der vorgängigen, aber nicht vorveröffentlichten Patentanmeldung gemäß EP-A 0 579 021 ist bekannt, daß sich bestimmte Halogenaromaten herstellen lassen , indem man Halogenameisensäureester in Gegenwart von bestimmten Katalysatoren in der Gasphase decarboxyliert. Entsprechende Umsetzungen in flüssiger Phase werden aber nicht erwähnt.

Schließlich geht aus der FR-A 2 647 107 hervor, daß man Fluoraromaten erhält , wenn man Halogenameisensäure-arylester in Gegenwart von Fluorwasserstoff und Lewis-Säuren in flüssiger Phase erhitzt. Die alleinige Verwendung von Fluorwasserstoff oder von Lewis-Säuren zur Durchführung entsprechender Reaktionen in flüssiger Phase wird jedoch nicht beschrieben.

Es wurde nun ein Verfahren zur Herstellung von Halogenaromaten der Formel 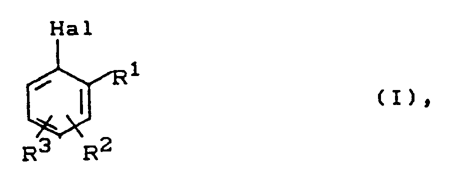 in der
- Hal: für Fluor oder Chlor,
- R¹: für C₁-C₆-Alkyl,
- R²: für Wasserstoff oder C₁-C₆-Alkyl und
- R³: für Wasserstoff, C₁-C₆-Alkyl, Fluor, Chlor oder Brom stehen,
gefunden,
das dadurch gekennzeichnet ist, daß man Halogenameisensäureester der Formel 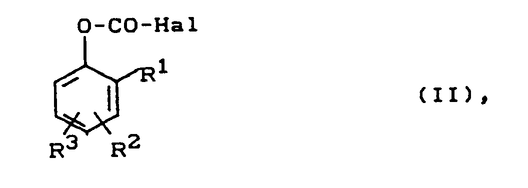 in der die verwendeten Symbole die bei Formel (I) angegebene Bedeutung haben,
in Gegenwart von entweder
- Fluorwasserstoff oder
- einer katalytischen Menge einer oder mehrerer Lewis-Säuren aus der Gruppe, bestehend aus Aluminiumhalogeniden, Eisenhalogeniden und Antimonhalogeniden,
und in flüssiger Phase auf 80 bis 280°C erhitzt.

Vorzugsweise steht in den Formeln (I) und (II)
- R¹ und R²: unabhängig voneinander für Methyl, Ethyl, n-Butyl oder i-Propyl und
- R³: für Wasserstoff oder Methyl.

Es ist weiterhin bevorzugt, daß der Substituent R² nicht für Wasserstoff steht und die Substituenten R¹ und R² in 2- und 6-Position in Bezug auf Hal vorliegen. In diesen Fällen werden Halogenaromaten der Formel (I) in besonders hohen Ausbeuten erhalten.

Das erfindungsgemäße Verfahren ist besonders für den Einsatz von 2,6-Dimethylphenylhalogenameisensäureestern geeignet, wobei man vorzugsweise aus dem Chlorameisensäureester mit den Lewis-Säuren 2,6-Dimethylchlorbenzol oder mit Fluorwasserstoff 2,6-Dimethylfluorbenzol herstellt.

Die zur Durchführung des erfindungsgemäßen Verfahrens als Ausgangsverbindungen benötigten Halogenameisensäureester der Formel (II) sind bekannt oder können analog zu bekannten Verbindungen hergestellt werden.

Soweit man das erfindungsgemäße Verfahren mit Fluorwasserstoff durchführt kommt dafür insbesondere handelsüblicher wasserfreier Fluorwasserstoff in Frage. Fluorwasserstoff kann beispielsweise in Mengen von 1 bis 100 Mol, vorzugsweise 2 bis 50 Mol, pro Mol Halogenameisensäureester der Formel (II) eingesetzt werden. Beim Arbeiten mit Fluorwasserstoff ist durch eine entsprechende Druckhaltung dafür zu sorgen, daß er bei Reaktionstemperatur zumindest noch teilweise in flüssiger Phase vorliegt. Die Arbeitsweise mit Fluorwasserstoff eignet sich besonders für die Herstellung von Fluoraromaten der Formel (I) aus Fluor- oder Chlorameisensäureestern der Formel (II).

Soweit man das erfindungsgemäße Verfahren mit Lewis-Säuren aus der Gruppe, bestehend aus Aluminiumhalogeniden, Eisenhalogeniden und Antimonhalogeniden, durchführt, sind beispielsweise Aluminiumtrichlorid, Eisentrichlorid, mit Chlorwasserstoff oder Fluorwasserstoff vorbehandeltes Aluminiumoxid und Antimonpentafluorid geeignet. Zur Umsetzung von Chlorameisensäureestern zu Chloraromaten verwendet man vorteilhafterweise die Chlorid enthaltenden Lewis-Säuren, insbesondere Aluminiumtrichlorid, zur Umsetzung von Fluorameisensäureestern zu Fluoraromaten vorteilhafterweise die Fluorid enthaltenden Lewis-Säuren, insbesondere Antimonpentafluorid, oder Fluorwasserstoff.

Die Lewis-Säuren, soweit es sich nicht um Fluorwasserstoff handelt, können z.B. in Mengen von 0,1 bis 10 Mol-%, vorzugsweise 1 bis 7 Mol-%, bezogen auf den Halogenameisensäureester der Formel (II), eingesetzt werden.

Beim Arbeiten mit den genannten Lewis-Säuren kann man häufig so verfahren, daß man den eingesetzten Halogenameisensäureester der Formel (II) bei Normaldruck am Rückfluß sieden läßt. Man kann auch in Gegenwart von Lösungsmitteln arbeiten, z.B. in Gegenwart von halogenierten Kohlenwasserstoffen, muß dann aber häufig erhöhte Drucke bzw. geschlossene Gefäße anwenden, um bei Reaktionstemperatur das gesamte System im wesentlichen in flüssiger Phase halten zu können. Man kann auch beim Einsatz von Fluorwasserstoff in Gegenwart von Lösungsmitteln arbeiten, was häufig jedoch keinen besonderen Vorteil bringt, da überschüssiger Fluorwasserstoff selbst als Lösungsmittel fungieren kann.

Bevorzugte Reaktionstemperaturen sind solche von 90 bis 240°C.

Man kann das erfindungsgemäße Verfahren beispielsweise in Reaktionsgefäßen für Normaldruck oder in Rührautoklaven diskontinuierlich durchführen, man kann auch kontinuierlich, z.B. in einem mit Füllkörpern gefüllten, beheizbaren Reaktionsrohr arbeiten, indem man unter Druck einen Halogenameisensäureester der Formel (II) und Fluorwasserstoff über die Füllkörper herunterrieseln läßt. Geeignete Materialien für Reaktoren für das erfindungsgemäße Verfahren sind z.B. Edelstähle.

Die erfindungsgemäße Umsetzung ist im allgemeinen nach 1 bis 5 Stunden beendet. Man kann dann das Reaktionsgemisch z.B. wie folgt aufarbeiten:
Es wird abgekühlt, gegebenenfalls vorhandener Druck entspannt, gegebenenfalls vorhandener überschüssiger Fluorwasserstoff abdestilliert, das dann vorliegende Gemisch auf Wasser oder Eis ausgetragen, gegebenenfalls nach Zusatz eines mit Wasser nicht mischbaren organischen Lösungsmittels, z.B. Methylenchlorid, die organische Phase abgetrennt, getrocknet und gegebenenfalls im Vakuum destilliert.

Das erfindungsgemäße Verfahren hat eine Reihe von Vorteilen. Es gestattet die Herstellung von Halogenaromaten der Formel (I), insbesondere von 2,6-Dialkyl-chlor- und -fluorbenzolen, auf einfache Weise (einstufig) und in guten Ausbeuten. Es kann in einfachen Apparaturen und bei relativ niedrigen Temperaturen durchgeführt werden. Man benötigt nur ein Hilfsmittel (Fluorwasserstoff oder eine Lewis-Säure).

Es ist überraschend, daß beim erfindungsgemäßen Verfahren trotz der Gegenwart von Lewis-Säuren oder Fluorwasserstoff Friedel-Crafts-Reaktionen nicht oder höchstens in ganz untergeordnetem Maß ablaufen.

### Beispiele

### Beispiel 1

50 g 2,6-Dimethylphenylchlorameisensäureester wurden mit 0,8 g wasserfreiem Aluminiumchlorid versetzt und bei Normaldruck auf 200°C erhitzt. Ab 180°C trat eine Entwicklung von Kohlendioxid ein, die nach 3,5 Stunden beendet war. Danach wurde das Reaktionsgemisch abgekühlt, mit Wasser versetzt, die organische Phase abgetrennt, die wäßrige Phase mit Methylenchlorid extrahiert, die abgetrennte organische Phase und die Methylenchloridphase vereinigt, über Natriumsulfat getrocknet und im Vakuum destilliert. 2,6-Dimethylchlorbenzol wurde in einer Ausbeute von 64 % erhalten.

### Beispiel 2

50 g 2,6-Dimethylphenylchlorameisensäureester wurden mit 0,3 g wasserfreiem Aluminiumchlorid versetzt und bei Normaldruck auf 200°C erhitzt. Anschließend wurden innerhalb von 2 Stunden weitere 150 g 2,6-Dimethylphenylchlorameisensäureester und ein weiteres Gramm wasserfreies Aluminiumchlorid eindosiert und gleichzeitig das entstehende 2,6-Dimethylchlorbenzol abdestilliert. Die Umsetzung war nicht vollständig und es wurde ein Gemisch aus 2,6-Dimethylphenylchlorameisensäureester und 2,6-Dimethylchlorbenzol erhalten. Die destillative Trennung ergab eine Ausbeute an 2,6-Dimethylchlorbenzol von 74 % (bezogen auf den Umsatz).

### Beispiel 3

50 g 2,6-Dimethylphenylchlorameisensäureester wurden mit 2,1 g wasserfreiem Aluminiumbromid versetzt und bei Normaldruck auf 200°C erhitzt. Nach Beendigung der Entwicklung von Kohlendioxid wurde das Reaktionsgemisch abgekühlt und gaschromatografisch untersucht. Es enthielt 2,6-Dimethylchlorbenzol.

### Beispiel 4

50 g 2,6-Dimethylphenylchlorameisensäureester wurden mit 1,3 g wasserfreiem Eisen(III)-chlorid versetzt und bei Normaldruck auf 200°C erhitzt. Nach Beendigung der Entwicklung von Kohlendioxid wurde das Reaktionsgemisch wie in Beispiel 1 beschrieben aufgearbeitet. 2,6-Dimethylchlorbenzol wurde in einer Ausbeute von 40 % erhalten,

### Beispiel 5

50 g 2,4,6-Trimethylphenylchlorameisensäureester wurden mit 0,5 g wasserfreiem Aluminiumchlorid versetzt und bei Normaldruck 1,5 Stunden lang auf 180°C erhitzt. Danach wurde das Reaktionsgemisch abgekühlt und wie in Beispiel 1 beschrieben aufgearbeitet. 2,4,6-Trimethylchlorbenzol wurde in einer Ausbeute von 72 % erhalten.

### Beispiel 6

20 g 2,3-Dimethylphenylchlorameisensäureester wurden mit 0,4 g wasserfreiem Aluminiumchlorid versetzt und 4,5 Stunden lang bei Normaldruck auf 200°C erhitzt. Danach wurde das Reaktionsgemisch abgekühlt und wie in Beispiel 1 beschrieben aufgearbeitet. 2,3-Dimethylchlorbenzol wurde in einer Ausbeute von 18 % erhalten, was trotz des ungünstigen Substitutionsmusters beachtlich ist.

### Beispiel 7

15 g 2,4-Dimethylphenylchlorameisensäureester wurden mit 0,5 g wasserfreiem Aluminiumchlorid versetzt und 3 Stunden lang bei Normaldruck auf 200°C erhitzt. Die Reaktionsmischung wurde abgekühlt und wie in Beispiel 1 beschrieben aufgearbeitet. 2,4-Dimethylchlorbenzol wurde in einer Ausbeute von 20 % erhalten, was trotz des ungünstigen Substitutionsmusters beachtlich ist.

### Beispiel 8

25 g 2-Isopropylphenylchlorameisensäureester wurden mit 0,35 g wasserfreiem Aluminiumchlorid versetzt und 3 Stunden lang bei Normaldruck auf 200°C erhitzt. Die Reaktionsmischung wurde abgekühlt und wie in Beispiel 1 beschrieben aufgearbeitet. 2-Isopropylchlorbenzol wurde in einer Ausbeute von 20 % erhalten, was trotz des ungünstigen Substitutionsmusters beachtlich ist.

### Beispiel 9

In einem Laborautoklaven aus Edelstahl wurden bei 0° 2000 ml wasserfreier Fluorwasserstoff vorgelegt und 500 g 2,6-Dimethylphenylchlorameisensäureester zugetropft. Danach wurde der Autoklav verschlossen und 3 Stunden lang auf eine maximale Temperatur von 130°C erhitzt. Die sich entwickelnden Gase wurden bei einem Druck von 26 bar entspannt. Danach wurde der Autoklav auf Raumtemperatur abgekühlt und der überschüssige Fluorwasserstoff abdestilliert. Das verbleibende-Reaktionsgemisch wurde auf Eis gegossen, die organische Phase abgetrennt und im Vakuum destilliert. Es wurde 2,6-Dimethylfluorbenzol in einer Ausbeute von 69 % erhalten.

### Beispiel 10

In einem Laborautoklaven aus Edelstahl wurden bei 0°C 1000 ml wasserfreier Fluorwasserstoff vorgelegt und 500 g 2,6-Dimethylphenylchlorameisensäureester zugetropft. Die Durchführung der Reaktion und die Aufarbeitung erfolgte wie in Beispiel 9 beschrieben. Es wurde 2,6-Dimethylfluorbenzol in einer Ausbeute von 51 % erhalten.

### Beispiel 11

In einem Laborautoklaven aus Edelstahl wurden bei 0°C 500 ml wasserfreier Fluorwasserstoff vorgelegt und 1000 g 2,6-Dimethylphenylchlorameisensäureester zugetropft. Danach wurde der Autoklav verschlossen und 2 Stunden lang auf eine maximale Temperatur von 110°C erhitzt, wobei die entstehenden Gase bei einem Druck von 30 bar entspannt wurden. Die Aufarbeitung des Reaktionsgemisches erfolgte wie in Beispiel 9 beschrieben, Die Destillation ergab 2,6-Dimethylfluorbenzol in einer Ausbeute von 11 % und 2,6-Dimethylphenylfluorameisensäureester in einer Ausbeute von 68 %. Letzterer kann einem neuen Ansatz zur Herstellung von 2,6-Dimethylfluorbenzol zugefügt werden (siehe Beispiel 15).

### Beispiel 12

In einem Laborautoklaven aus Edelstahl wurden bei 0°C 500 ml wasserfreier Fluorwasserstoff vorgelegt und 150 g 2,6-Dimethylphenylchlorameisensäureester zugetropft. Der Autoklav wurde verschlossen und 1,5 Stunden lang auf eine maximale Temperatur von 140°C erhitzt, wobei die entstehenden Gase bei einem Druck von 26 bar entspannt wurden, Die Aufarbeitung erfolgte wie in Beispiel 9 beschrieben, Es wurde 2,6-Dimethylfluorbenzol in einer Ausbeute von 68,4 % erhalten.

### Beispiel 13

In einem 3 l fassenden Autoklaven wurden 1,1 kg wasserfreien Fluorwasserstoff vorgelegt, der Autoklav verschlossen und auf 130°C erhitzt. Bei dieser Temperatur wurde im Verlaufe von 3 Stunden 1 kg 2,6-Dimethylphenylchlorameisensäureester zugepumpt, wobei die entstehenden Gase bei einem Druck von 40 bar entspannt wurden, Abschließend wurde das Gemisch 1 Stunde bei 130°C nachgerührt. Danach wurde das Reaktionsgemisch abgekühlt und überschüssiger Fluorwasserstoff bei 100 mbar abdestilliert. Die weitere Aufarbeitung des Reaktionsgemisches erfolgte wie in Beispiel 9 beschrieben. 2,6-Dimethylfluorbenzol wurde in einer Ausbeute von 58,3 % erhalten.

### Beispiel 14

In einem Autoklaven aus Edelstahl wurden bei 0°C 70 ml Fluorwasserstoff und 200 ml 1,1,2-Trifluor-1,2,2-trichlorethan vorgelegt und 100 g 2,6-Dimethylphenylchlorameisensäureester zugetropft. Der Autoklav wurde verschlossen und 5 Stunden lang auf eine maximale Temperatur von 160°C erhitzt, wobei die entstehenden Gase bei einem Druck von 30 bar entspannt wurden. Die Aufarbeitung des Reaktionsgemisches erfolgte wie in Beispiel 9 beschrieben, 2,6-Dimethylfluorbenzol wurde in einer Ausbeute von 30 % und 2,6-Dimethylphenylfluorameisensäureester in einer Ausbeute von 40 % erhalten. Letzterer kann einem neuen Ansatz zur Herstellung von 2,6-Dimethylfluorbenzol zugefügt werden (siehe Beispiel 15).

### Beispiel 15

In einem Laborautoklaven aus Edelstahl wurden bei 0°C 200 ml wasserfreier Fluorwasserstoff vorgelegt und 100 g 2,6-Dimethylphenylfluorameisensäureester zugetropft. Der Autoklav wurde verschlossen und 1,5 Stunden lang auf eine maximale Temperatur von 140°C erhitzt. Die Aufarbeitung des Reaktionsgemisches erfolgte wie in Beispiel 9 beschrieben, 2,6-Dimethylfluorbenzol wurde in einer Ausbeute von 75 % erhalten.

### Beispiel 16

100 g 2,6-Dimethylphenylfluorameisensäureester wurden mit 3,9 g Antimonpentafluorid versetzt und 5 Stunden lang bei Normaldruck auf 200°C erhitzt. Danach wurde das Reaktionsgemisch abgekühlt und wie in Beispiel 1 beschrieben aufgearbeitet. 2,6-Dimethylfluorbenzol wurde in einer Ausbeute von 40 % erhalten.

### Beispiel 17

In einem Laborautoklaven aus Edelstahl wurden bei 0°C 600 ml wasserfreier Fluorwasserstoff vorgelegt und 180 g 2,3-Dimethylphenylchlorameisensäureester zugetropft. Danach wurde der Autoklav verschlossen und 4 Stunden auf eine maximale Temperatur von 140°C erhitzt. Die Aufarbeitung des Reaktionsgemisches erfolgte wie in Beispiel 9 beschrieben. Es wurde ein Gemisch aus 43 % 2,3-Dimethylfluorbenzol, 3,8 % Trimethylfluorbenzol und 2,8 % 2,6-Dimethylfluorbenzol erhalten.

### Beispiel 18

In einem Laborautoklaven aus Edelstahl wurden bei 0°C 600 ml wasserfreier Fluorwasserstoff vorgelegt und 150 g 2,4,6-Trimethylphenylchlorameisensäureester zugetropft. Danach wurde der Autoklav verschlossen und 3 Stunden lang auf eine maximale Temperatur von 110°C erhitzt. Die Aufarbeitung des Reaktionsgemisches erfolgte wie in Beispiel 9 beschrieben. Es wurde ein Gemisch aus 43 % 2,4,6-Trimethylfluorbenzol, 23 % Trimethylfluorbenzol (Isomer), 15 % Tetramethylfluorbenzol und 9,8 % Dimethylfluorbenzol erhalten.

### Beispiel 19

Ein rohrförmiger beheizbarer` 0,7 l fassender Edelstahlreaktor wurde mit Edelstahl-Maschendrahtfüllkörpern mit einem Durchmesser und einer Höhe von 6 mm und einer Maschendichte von 3600 Maschen/cm² gefüllt. Der Reaktor wurde so beheizt, daß sich im unteren Drittel des Reaktionsraumes eine Temperatur von 120°C einstellte. In diesen Reaktor wurden bei einem Druck von 23 bar pro Stunde 18,5 g 2,6-Dimethylphenylchlorameisensäureester und 200 g wasserfreier Fluorwasserstoff von oben eingespeist und durch den Reaktor rieseln gelassen. Die Druckhaltung erfolgte am unteren Auslaß des Reaktors mit Hilfe eines Ventils.

Das im Verlaufe von 2 Stunden den Reaktor verlassende Reaktionsgemisch wurde in einer Mischung aus 500 g Eis und 200 ml Methylenchlorid aufgefangen. Die organische Phase wurde danach abgetrennt, die wäßrige Phase mit 200 ml Methylenchlorid nachgewaschen, die Methylenchloridphasen vereinigt, durch Zusatz von Natriumfluorid noch vorhandener Fluorwasserstoff entfernt, danach über Natriumsulfat/Natriumfluorid getrocknet und gaschromatografisch untersucht. Bei einem Umsatz von 100 % hatte sich mit einer Selektivität von 88 % 2,6-Dimethylfluorbenzol gebildet.

## Patentansprüche

1. Verfahren zur Herstellung von Halogenaromaten der Formel 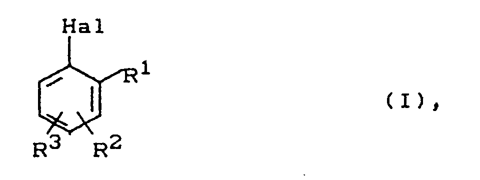 in der
Hal für Fluor oder Chlor,
R¹ für C₁-C₆-Alkyl,
R² für Wasserstoff oder C₁-C₆-Alkyl und
R³ für Wasserstoff, C₁-C₆-Alkyl, Fluor, Chlor oder Brom stehen,
dadurch gekennzeichnet, daß man Halogenameisensäureester der Formel 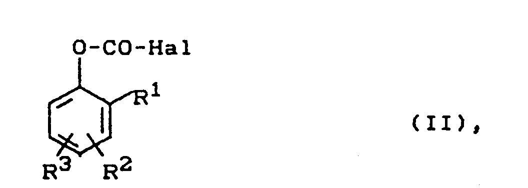 in der die verwendeten Symbole die bei Formel (I) angegebene Bedeutung haben,
in Gegenwart von entweder
- Fluorwasserstoff oder
- einer katalytischen Menge einer oder mehrerer Lewis-Säuren aus der Gruppe , bestehend aus Aluminiumhalogeniden, Eisenhalogeniden und Antimonhalogeniden,
und in flüssiger Phase auf 80 bis 280°C erhitzt.

2. Verfahren nach Anspruch 1 , dadurch gekennzeichnet, daß bei den Formeln (I) und (II) der Substituent R² nicht für Wasserstoff steht und die Substituenten R¹ und R² in 2- und 6-Position in Bezug auf Hal vorliegen.

3. Verfahren nach Ansprüchen 1 und 2, dadurch gekennzeichnet, daß man pro Mol Halogenameisensäureester der Formel (II) 1 bis 100 Mol Fluorwasserstoff oder 0,1 bis 10 Mol-% Lewis-Säuren aus der Gruppe, bestehend aus Aluminiumhalogeniden, Eisenhalogeniden und Antimonhalogeniden , einsetzt.

4. Verfahren nach Ansprüchen 1 bis 3 , dadurch gekennzeichnet, daß man beim Arbeiten mit Lewis-Säuren den eingesetzten Halogenameisensäureester der Formel (II) bei Normaldruck am Rückfluß sieden läßt.

5. Verfahren nach Ansprüchen 1 bis 4, dadurch gekennzeichnet, daß man bei 90 bis 240°C arbeitet.

6. Verfahren nach Ansprüchen 1 bis 5, dadurch gekennzeichnet, daß man nach Beendigung der Reaktion das Reaktionsgemisch abkühlt, gegebenenfalls vorhandenen Druck entspannt, gegebenenfalls vorhandenen überschüssigen Fluorwasserstoff abdestilliert, das dann vorliegende Gemisch auf Wasser oder Eis austrägt, gegebenenfalls nach Zusatz eines mit Wasser nicht mischbaren organischen Lösungsmittels die organische Phase abtrennt, trocknet und, gegebenenfalls im Vakuum, destilliert.

7. Verfahren nach Ansprüchen 1 bis 6, dadurch gekennzeichnet, daß man 2,6-Dimethylphenylhalogenameisensäureester einsetzt.

8. Verfahren nach Anspruch 7, dadurch gekennzeichnet, daß man 2,6-Dimethylphenylchlorameisensäureester und eine der genannten Lewis-Säuren einsetzt.

9. Verfahren nach Anspruch 7, dadurch gekennzeichnet, daß man 2,6-Dimethylchlorameisensäureester und Fluorwasserstoff einsetzt .

## Claims

1. Process for the preparation of halogenated aromatics of formula 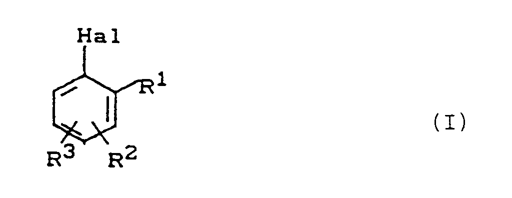 in which
Hal is fluorine or chlorine,
R¹ is C₁-C₆-alkyl,
R² is hydrogen or C₁-C₆-alkyl and
R³ is hydrogen, C₁-C₆-alkyl, fluorine, chlorine or bromine,
characterised in that halogenoformic acid esters of formula 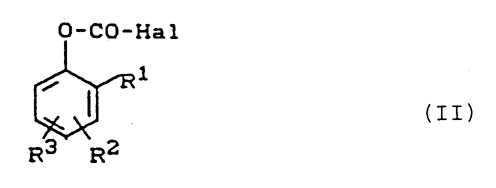 in which the symbols used are as defined for formula (I),
are heated to 80 to 280°C
in the presence of either
- hydrogen fluoride or
- a catalytic amount of one or more Lewis acids from the group comprising aluminium halides, iron halides and antimony halides,
and in the liquid phase.

2. Process according to Claim 1, characterised in that, in formulae (I) and (II), the substituent R² is not hydrogen and the substituents R¹ and R² are located in the 2- and 6-positions relative to Hal.

3. Process according to Claims 1 and 2, characterised in that 1 to 100 mol of hydrogen fluoride, or 0.1 to 10 mol% of Lewis acids from the group comprising aluminium halides, iron halides and antimony halides, are used per mole of halogenoformic acid ester of formula (II).

4. Process according to Claims 1 to 3, characterised in that, when working with Lewis acids, the halogenoformic acid ester of formula (II) used is refluxed, at normal pressure.

5. Process according to Claims 1 to 4, characterised in that the reaction is carried out at 90 to 240°C.

6. Process according to Claims 1 to 5, characterised in that, after the reaction has ended, the reaction mixture is cooled, any remaining pressure is released, any excess hydrogen fluoride present is distilled off, the resulting mixture is discharged onto water or ice and, optionally after the addition of a water-immiscible organic solvent, the organic phase is separated off, dried and distilled, optionally under vacuum.

7. Process according to Claims 1 to 6, characterised in that 2,6-dimethylphenyl halogenoformate is used.

8. Process according to Claim 7, characterised in that 2,6-dimethylphenyl chloroformate and one of the Lewis acids mentioned are used.

9. Process according to Claim 7, characterised in that 2,6-dimethyl chloroformate and hydrogen fluoride are used.

## Revendications

1. Procédé pour la préparation de composés aromatiques halogénés répondant à la formule 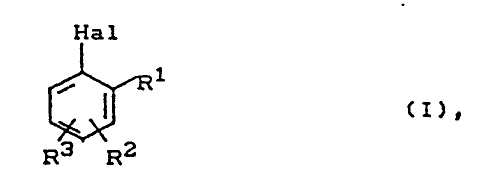 dans laquelle
Hal représente un atome de fluor ou un atome de chlore,
R¹ représente un groupe alkyle en C₁-C₆,
R² représente un atome d'hydrogène ou un groupe alkyle en C₁-C₆, et
R³ représente un atome d'hydrogène, un groupe alkyle en C₁-C₆, un atome de fluor, un atome de chlore ou un atome de brome,
caractérisé en ce qu'on chauffe des esters halogénoformiques répondant à la formule 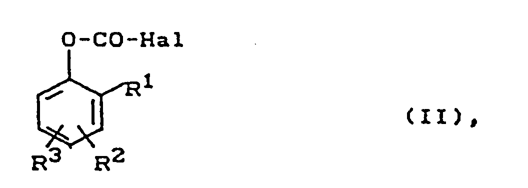 dans laquelle les symboles utilisés ont la signification indiquée à la formule (I)
en présence
- soit d'acide fluorhydrique,
- soit d'une quantité catalytique d'un ou de plusieurs acides de Lewis choisis parmi le groupe constitué par des halogénures d'aluminium, des halogénures du fer et des halogénures d'antimoine,
et en phase liquide à une température de 80 à 280°C.

2. Procédé selon la revendication 1, caractérisé en ce que, dans les formules (I) et (II), le substituant R² ne représente pas un atome d'hydrogène et les substituants R¹ et R² sont présents en position 2 et en position 6 par rapport à Hal.

3. Procédé selon les revendications 1 et 2, caractérisé en ce qu'on met en oeuvre, par mole d'esters halogénoformiques de formule (II), de 1 à 100 moles d'acide fluorhydrique ou de 0,1 à 10 moles % d'acides de Lewis choisis parmi le groupe constitué par des halogénures d'aluminium, des halogénures du fer et des halogénures d'antimoine.

4. Procédé selon les revendications 1 à 3, caractérisé en ce que, lorsqu'on travaille avec des acides de Lewis, on porte à ébullition au reflux sous pression normale les esters halogénoformiques de formule (II) mis en oeuvre.

5. Procédé selon les revendications 1 à 4, caractérisé en ce qu'on travaille à une température de 90 à 240°C.

6. Procédé selon les revendications 1 à 5, caractérisé en ce que, une fois que la réaction est arrivée à son terme, on refroidit le mélange réactionnel, on détend la pression éventuellement présente, on sépare par filtration l'acide fluorhydrique en excès éventuellement présent, on déverse sur de l'eau ou de la glace le mélange que l'on obtient en l'occurrence, on sépare la phase organique le cas échéant après addition d'un solvant organique non miscible à l'eau, on sèche et on distille, le cas échéant sous vide.

7. Procédé selon les revendications 1 à 6, caractérisé en ce qu'on met en oeuvre l'ester 2,6-diméthylphénylhalogénoformique.

8. Procédé selon la revendication 7, caractérisé en ce qu'on met en oeuvre l'ester 2,6-diméthylphénylchloroformique et un des acides de Lewis mentionnés.

9. Procédé selon la revendication 7, caractérisé en ce qu'on met en oeuvre l'ester 2,6-diméthylchloroformique et l'acide fluorhydrique.
